# EUROPÄISCHE PATENTSCHRIFT

(11) **EP 1 744 837 B1**
(45) Veröffentlichungstag und Bekanntmachung des Hinweises auf die Patenterteilung: **24.10.2007**
(21) Anmeldenummer: 05735319.5
(22) Anmeldetag: 23.04.2005
(51) Int. Cl.: B06B 1/06, H01L 41/047

(54) **ULTRASCHALLWANDLER SOWIE VERFAHREN ZUR HERSTELLUNG DESSELBEN**
ULTRASOUND TRANSDUCER AND METHOD FOR PRODUCING THE SAME
TRANSDUCTEUR D'ULTRASONS ET SON PROCEDE DE FABRICATION

(30) Priorität: 08.05.2004 DE 102004022838
(43) Veröffentlichungstag der Anmeldung: 24.01.2007
(73) Patentinhaber: Forschungszentrum Karlsruhe GmbH, 76133 Karlsruhe (DE)
(72) Erfinder: SCHLOTE-HOLUBEK, Klaus, 76199 Karlsruhe (DE); GÖBEL, Georg, 76137 Karlsruhe (DE); STOTZKA, Rainer, 76139 Karlsruhe (DE)
(86) Internationale Anmeldenummer: PCT/EP2005/004389
(87) Internationale Veröffentlichungsnummer: WO 2005/107962

(56) Entgegenhaltungen:
- EP-A- 1 145 772
- WO-A-02/40184
- WO-A-03/047770

## Beschreibung

Die vorliegende Erfindung betrifft einen Ultraschallwandler, insbesondere Wandlerarrays, die aus einer Anzahl von Wandlerelementen bestehen, gemäß des ersten sowie ein Verfahren zur Herstellung eines Ultraschallwandlers gemäß des neunten Patentanspruchs.

Wie in vielen Bereichen der Elektronik und der Sensorik werden auch in der Ultraschalltechnik die Komponenten für Geräten und Systemen immer kleiner und leistungsfähiger. Wichtiger Bestandteil solcher Systeme sind Ultraschallsensoren die in den meisten Fällen speziell auf ein Messproblem zugeschnitten sind. Kosten und Qualität dieser Komponenten stehen bei der Entwicklung von Ultraschallsystemen gleichermaßen im Vordergrund. Es ist unverzichtbar, dass die Schallwandler möglichst gleiche akustische und elektrische Eigenschaften aufweisen.

Ein Beispiel für den hohen Entwicklungsstand der Ultraschallprüftechnik sind Ultraschall-Wandlerarrays oder auch Gruppenstrahler. Ein Wandlerarray besteht aus einer Anzahl von Einzelwandlerelementen, vorzugsweise Säulen oder Streifen, die aus physikalischen Gründen sehr klein und dicht nebeneinander angeordnet sind um z.B. durch eine phasenversetzte Ansteuerung der einzelnen Wandlerelemente oder Gruppen dieser das akustische Signal in die gewünschte Richtung ablenken. Die Größe und Abstände solcher Einzelwandlerelemente liegen bei einer Frequenz von 5 MHz unter 0.5 mm. Mit solchen Anordnungen ist es möglich einen akustischen Schwenk durch das zu prüfende Medium durchzuführen, ohne die Position des Ultraschallmesskopfes zu ändern. Der Aufbau kann dann in einer Linear- oder in einer Matrix-Struktur erfolgen. Diese Art von Ultraschallsensoren findet man oft in der Werkstoffprüfung und Medizintechnik.

[2] offenbart beispielhaft mit der Ultraschall-Computertomographie (USCT) für die Brustkrebsfrüherkennung eine spezielle Anwendung für eingangs genannte Ultraschallwandler der jüngsten Generation. Für ein derartiges System ist eine hohe Anzahl von Ultraschallwandler geringer Größe (z.B. 1 x 1 mm) erforderlich, welche sich näherungsweise wie einzelne punktförmige Strahler verhalten. Sie werden um ein Prüfvolumen herum als Matrixstruktur zylinderförmig oder in Form einer Halbkugel zueinander angeordnet, womit räumliche Informationen (3D) aus dem zu untersuchenden Volumen ohne eine mechanische Bewegung eines der Ultraschallwandler erfassbar sind. Diese Messanordnung, verbunden mit einer parallelen Signalverarbeitung eignet sich für die vollständige tomographische Erfassung eines Körperteils eines Patienten bereits mit Messzeiten kleiner 0,05 s, wodurch die Bedeutung einer Chronologie von Einzelaufnahmen nahezu vernachlässigbar wird. Die hohe Anzahl (mehrere Tausend) der Ultraschallwandler geringer Größe entspricht dabei prinzipiell einem zylinderförmigen oder kugelsegmentförmigen Ultraschallwandlerarray, mit den einzelnen Ultraschallwandler als Wandlerelemente.

Die akustischen Eigenschaften sind physikalisch unmittelbar an die geometrischen Daten von Ultraschallwandlern, insbesondere von Ankopplungsschicht und Wandlerelement (meist piezoelektrischer Körper) gekoppelt. Fertigungsungenauigkeiten, auch Toleranzbreiten bei der Herstellung von Ultraschallwandler beeinflussen somit Exaktheit und Toleranzbreite von akustischen Eigenschaften einer Ultraschallwandlerbauserie. Auch wenn geringe Abweichungen dieser akustischen Eigenschafen innerhalb einer gewissen Bandbreite auf elektronischem Wege kompensierbar oder in einer anschließenden Qualitätskontrolle eingrenzbar sind, so ist es auch aus ökonomischen Gesichtspunkten unverzichtbar, dass Ultraschallwandler die vorbestimmten akustischen und elektrischen Eigenschaften mit geringen Toleranzbreiten auch aufweisen.

Die Ankopplungs- oder Anpassungsschicht dient der möglichst verlustarmen Ankopplung eines Wandlerelements an ein Medium. Sie gleicht die unterschiedlichen akustischen Impedanzen von Wandler und Wasser aus, wobei die Dicke dieser Schicht idealerweise 1/4 der Wellenlänge der Dickenresonanzfrequenz des piezoelektrischen Körpers (Wandler) beträgt. Derartige Schichten werden üblicherweise aus entsprechenden Vergussmassen hergestellt, welche im Rahmen einer Fertigung auf das bereits kontaktierte und elektrisch angeschlossene Wandlerelement aufgebracht werden. Es folgt üblicherweise eine Anpassung der Ankopplungsschichtdicke auf die Frequenz (Wellenlänge) des Wandlerelements mittels einer mechanischen und damit aufwendigen Nachbearbeitung der Ankopplungsschicht. Der Aufbau der meisten Ultraschallwandler erfolgt demnach meist von innen nach außen, d. h. beginnend mit dem Wandlerelement.

In [1] ist vor dem Hintergrund einer einfachen Herstellbarkeit ein Ultraschallwandler und Verfahren zur Herstellung eines Ultraschallwandlers beschrieben. In Abstrahlungsrichtung weist ein piezoelektrische Körper (Wandlerelement) eine flächige Kontaktierung auf, auf die wiederum eine Ankopplungsschicht aufgebaut ist. Die Kontaktierung auf der gegenüberliegenden Elektrodenfläche des Körpers erfolgt dagegen über eine aufgeklebte oder angepresste Leiterbahnfolie. Die Pressung erfolgt beispielsweise mechanisch über ein Anpressen des Dämpfungskörpers. Über die Anordnung der Leiterbahnen auf der Leiterbahnfolie sind einzelne Bereiche des Körpers oder des Wandlers selektiv ansteuerbar. Die abstrahlungsseitige Elektrode weist selbst keine Leiterbahnstruktur auf.

Ein derartiger Ultraschallwandler benötigt nach wie vor eine Ankopplungsschicht, die als separates Bauteil auf die abstrahlungsseitige Elektrode aufgesetzt wird. Zudem werden die Anschlüsse zu den beiden Elektroden über einzelne Drahtverbindungen hergestellt, was einen vergleichsweise hohen Fertigungsaufwand erfordert.

In [3] wird ein Ultraschall-Wandlersystem beschrieben, bei dem mehrere piezokeramischer Körper mit jeweils einer Elektrodenfläche flächig und direkt auf eine Leiterplatte aufgeklebt sind. Die Anschlüsse zu den Elektroden zu einer Sende- und Empfangselektronik auf benachbarten Leiterplatten erfolgt mittels gebondeter Drähte.

Ausgehend davon ist es Aufgabe der Erfindung, einen Ultraschallwandler vorzuschlagen, welcher einerseits eine weitere Vereinfachung der Herstellbarkeit allgemein beinhaltet, insbesondere dabei die Maßhaltigkeit und Qualität der Ankopplungsschicht mit geringen Toleranzbreiten noch weiter verbessert.

Die Aufgabe wird mit einem Ultraschallwandler gemäß des ersten Patentanspruchs sowie mit einem verfahren zur Herstellung desselben gemäß des neunten Patentanspruchs gelöst. Die abhängigen Unteransprüche geben vorteilhafte Ausführungsformen wieder.

Der wesentliche Grundgedanke der Erfindung besteht darin, einen piezoelektrischen Körper mit einer der beiden planparallel zueinander angeordneten Elektrodenfläche auf Abstandshaltern in einfacher Weise exakt ausgerichtet, elektrisch kontaktierend, planparallel und reproduzierbar auf eine Platine zu positionieren und zu fixieren. Auf der Platine befindet sich zu diesem Zweck eine aus einer flächigen Platinenbeschichtung herausgeätzte oder über eine Dickfilmtechnik (z.B. mit Siebdruck) aufgebrachte Leiterbahnstruktur. Diese dient einerseits als elektrische Kontaktierung mit der Elektrodenfläche, andererseits mit ihrer exakten und konstanten Leiterbahnhöhe als Abstandshalter zwischen Platine und piezoelektrischen Körper, und zwar über den gesamten Elektrodenbereich erstreckend. Zur Fixierung des piezoelektrischen Körpers auf der Platine eignen sich Lote oder Klebstoffe, welche bei einem Zusammenpressen von piezoelektrischen Körper und Platine lokal in die neben den Leiterbahnstruktur verbleibenden Hohlräume zwischen Platine und dem Ultraschallwandler ausweichen und diese dabei vollständig ausfüllen. Das Aufbringen des piezoelektrischen Körpers erfolgt durch ein Anpressen dieses auf die Leiterbahnstruktur, wobei der Klebstoff oder das Lot an mindestens einer Stelle durch das Aufeinandertreffen der Leiterbahnstruktur und der Elektrodenfläche durchkontaktiert wird. Die Leiterbahnstruktur dient damit gleichzeitig als elektrischer Anschluss für die abstrahlungsseitige Elektrodenfläche.

Die Dicke der Ankopplungsschicht beträgt idealer Weise λ/4, wobei die Ankopplungsschicht die Platine umfasst.

Vorzugsweise erstreckt sich die Leiterbahnstruktur nur unter Bereiche des piezoelektrischen Körpers, welche akustisch vergleichsweise wenig oder nicht aktiv sind, beispielsweise auf die unmittelbaren Randbereiche des piezoelektrischen Körpers oder auf die Bereiche, in denen der piezoelektrische Körper nicht zur Erzeugung oder Empfang eines Ultraschallimpuls herangezogen wird. Wenig aktiv heißt in diesem Zusammenhang, dass durch diese Bereiche die Ultraschallabstrahlungscharakteristik des Ultraschallwandlers zwar messbar ist, diese aber eine Ultraschallprüfung praktisch nicht beeinflussen oder beeinträchtigen. Bevorzugt wird der piezoelektrische Körper in diesen Bereichen nicht durch die Sende- oder Empfangselektronik angesteuert, wobei es sich anbietet, diese Bereiche von der Erstreckung der oberen Elektrode auszuschließen. Die Gesamtfläche dieser Bereiche entspricht maximal 10 % der Überdeckung durch den piezoelektrischen Körper. Vorzugsweise sind die Leiterbahnstrukturen zur Vermeidung von Kippeleffekten so gestaltet, dass eine statisch bestimmte Auflage für die untere Elektrode des piezoelektrischen Körpers entsteht, beispielsweise über drei Auflagerpunkte. Kleine Auflagerpunkte bewirken beim Anpressen des piezoelektrischen Körpers in diesem ein hohes Flächenpressungsniveau, d. h. Spannungsspitzen, aber auch in vorteilhafter Weise eine höhere Zuverlässigkeit bei Kontaktierung der Elektrode über die Auflagerpunkte.

Eine Güte der Kontaktierung lässt sich beispielsweise durch eine Messung des ohmschen Widerstands zwischen zwei als Abstandshalter dienenden und durch die untere Elektrode überbrückten Leiterbahnen überprüfen.

Das erforderliche Eigenschaftsprofil des Platinenmaterials wird insbesondere durch die gute Schallübertragung vom piezoelektrischen Körper auf ein Ankopplungsmedium, in das der Ultraschallimpuls eingespeist wird, vorgegeben. Ferner muss das Platinenmaterial sich zum Ankopplungsmedium, vorzugsweise wässrige Lösungen oder ein in der Medizintechnik verwendetes Ultraschallankopplungsgel, inert verhalten und darf dieses nicht in sich Aufnehmen oder Anbinden. Eine für die Schallausbreitung in einem Werkstoff wichtigste Materialkenngröße ist die spezifische akustische Impedanz Z, definiert als Produkt von Schallgeschwindigkeit c und Dichte ρ eines Materials. Einen vollständigen verlustfreien Schallübergang (Transmission) an einer Grenzfläche erhält man, wenn die Medien auf beiden Seiten der Grenzfläche die gleiche akustische Impedanz aufweisen, während mit dem Impedanzunterschied der an der Grenzfläche reflektierende Anteil zuungunsten der Transmission eines Ultraschallsignals zunimmt. Der Unterschied der akustischen Impedanzen zweier Materialien oder Medien wird daher als Maß für die Übertragungsverluste bei einem Materialübergang von einem zum anderen dieser Materialien / Medien herangezogen.

Als Platinen eignen sich insbesondere handelsübliche Platinen für Mikrowellen- oder Hochfrequenzanwendungen aus einem homogenen Kunststoff-Keramik-Verbund (d.h. z.B. ohne Gewebeeinlagen), welche nicht nur die zuvor genannten Materialanforderungen einer Ankopplungsschicht erfüllen, sondern in vorteilhafter Weise als Industrieprodukt mit homogener und gleich bleibender Materialgüte bei exakter Geometrie reproduzierbar und preisgünstig verfügbar sind. Diese Platinen haben den großen Vorteil, dass sie in vielen Dicken kommerziell erhältlich sind und die erforderlichen mechanischen und chemischen Eigenschaften besitzen.

Die Leiterbahnhöhe bestimmt den Abstand zwischen Platine und unterer Elektrode und damit die Klebstoff- oder Lotdicke. Je nach Dicke dieser Zwischenlage, üblich sind Dicken zwischen 20 und 70 µm (d.h. unter λ/4 bei 10 MHz in der Platine), ist sie ähnlich der metallischen Elektroden des piezokeramischen Körpers (Wandlers) vernachlässigbar oder muss ab einer gewissen Dicke in die Berechnung der Ankopplungsschicht berücksichtigt werden.

Es liegt im Rahmen der Erfindung, die Materialeigenschaften wie die akustische Impedanz der eingesetzten Materialien insbesondere von Platine und Kleber durch geeignete Maßnahmen wie durch Einmischung von Substanzen oder Pulvern anzupassen. Aufgrund der hohen akustischen Impedanzwerte von Loten ist dies hier nicht möglich. Lote eignen sich daher bevorzugt aus vorgenanten Gründen nur bei geringen Leiterbahnhöhen.

Als piezoelektrischer Körper eignen sich sowohl monolithische unstrukturierte Körper wie auch Wandler, welche sich aus einer Vielzahl einzelner Wandlerelemente zu einem Wandlerarray zusammensetzen. Insbesondere beim zuletzt genannten bietet die Erfindung wesentliche Verbesserungen der Herstellbarkeit, da ein zunächst unstrukturierter piezoelektrischer Körper mit der Platine verbunden und im Anschluss daran strukturiert, d. h. durch ein geeignetes Verfahren wie Sägen oder Ultraschallzerspanung in die einzelnen Wandlerelemente segmentiert werden kann. Die Platine bietet dabei in vorteilhafter Weise nicht nur eine geeignete Einspannungsmöglichkeit für den vorgenannten Herstellungsprozess, sondern stabilisiert bei einer Segmentierung auch die Einzelnen Wandlerelemente auf der Platine.

Im Anschluss einer Segmentierung oder Strukturierung bietet es sich an, die hierdurch entstandenen Hohlräume im Bereich des Körpers oder des Wandlers durch geeignete Stoffe wie Kunststoffe wieder aufzufüllen, wobei in einer möglichen Ausführungsform diese Stoffe gleichzeitig für eine Klebverbindung für aufgesetzte Komponenten wie der Dämpfungskörper heranziehbar sind.

In vorgenannter Weise lässt sich auch der eingangs im Rahmen des Stands der Technik erwähnte Dämpfungskörper auf der oberen zweiten Elektrodenfläche durch eine Platine mit einer Leiterbahnstruktur vorgegebener Höhe ersetzen.

Alternativ können die vorgenannten Hohlräume im Bereich des Körpers oder des Wandlers auch ungefüllt verbleiben, insbesondere dann, wenn die mit den Stoffen verbundene erhöhte Dämpfung des Ultraschallwandlers unerwünscht ist. Dabei bietet es sich auch optional an, einen Dämpfungskörper auf den segmentierten Wandler oder den strukturierten Körper in vorgenannter Weise zu befestigen, wobei die einzelnen Wandlerelemente auf beiden Elektrodenflächen fixiert sind.

Die Erfindung wird anhand von Ausführungsbeispielen näher erläutert. Es zeigen
Fig. 1 den prinzipiellen Aufbau eines Ultraschallwandlers mit den Komponenten gemäß des Stands der Technik,
Fig. 2 den prinzipiellen Aufbau einer Ausführungsform mit monolithischem piezoelektrischen Körper,
Fig. 3 den prinzipiellen Aufbau einer Ausführungsform mit einem Wandler, welcher sich aus einer Vielzahl von Wandlerelementen zusammensetzt,
Fig. 4 eine beispielhafte Gestaltung der Platine in Draufsicht,
Fig. 5 und 6 zwei weitere beispielhafte Gestaltungen der Platine mit aufgesetzten Wandlern sowie
Fig. 7 und 8 zwei beispielhafte Gestaltungen der Platine mit aufgesetzten Wandlern für medizinische Anwendungen.

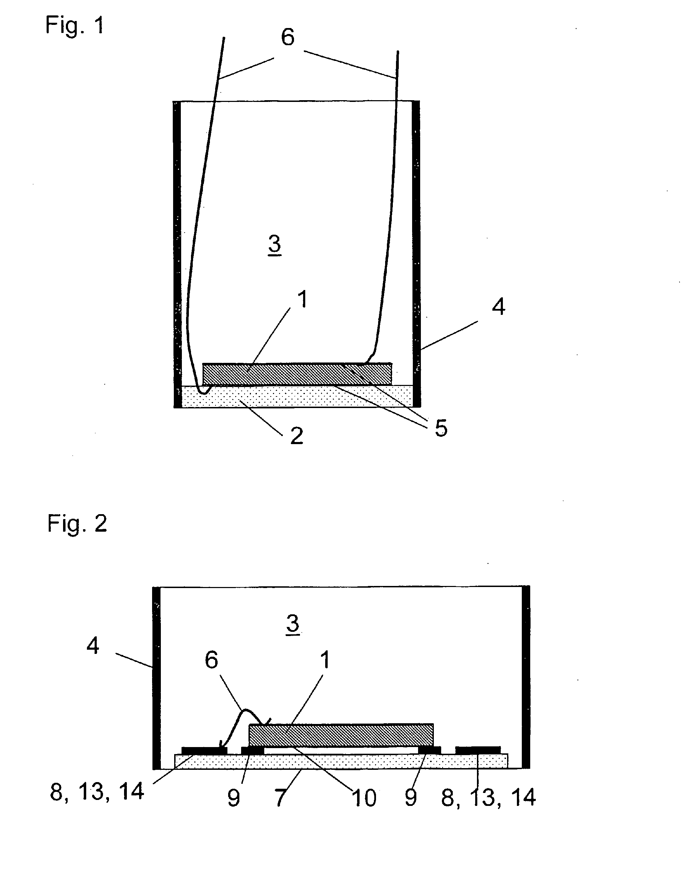

Fig. 1 gibt die Grundkomponenten eines Ultraschallwandlers üblicher Bauart wieder, nämlich einen piezoelektrischen Körper, dem Wandler 1, welcher fest mit einer Ankopplungsschicht 2 verbunden ist, deren Dicke idealer weise 1/4 der Wellenlänge λ beträgt und das Ende eines Gehäuses 4 abschließt. Im Gehäuse befindet sich ein rückseitiger Dämpfungskörper 3, welcher im vorliegenden Fall als Vergussmasse im Gehäuse gebildet wird. Die beiden Elektroden 5 des Wandlers 1 über kabelförmige elektrische Anschlüsse 6 an eine nicht dargestellte Steuer- und Messeinheit angeschlossen. Wie eingangs erläutert, wird Dicke der Ankopplungsschicht durch eine aufwendige Nachbearbeitung eingestellt.

Um diese Probleme zu vermeiden wird eine HF-Platine der vorgenannten Art als Anpassungsschicht und Träger für den weiteren Aufbau verwendet. Auf diese Platine wird dann ein piezoelektrischer Wandler aufgeklebt. Die im Rahmen des Ausführungsbeispiels verwendete Platine eignet sich für Ultraschallwandlersysteme bis zu einer Frequenz von ca. 10 MHz, liegt mit einer akustischen Impedanz von ca. 6 bis 8 MRayl genau zwischen der von üblichen PZT-Werkstoffen ca. 20 bis 35 MRayl und von Wasser bzw. wässrigen Lösungen ca. 1,5 MRayl. Die Platinen sind in Dicken von ca. 0,1 bis 1,5 mm kommerziell erhältlich. Ausgehend von einer Ankopplungsschichtdicke der genannten λ/4 eignen sich die genannten kommerziellen Platinen für die Herstellung von Ultraschallwandlern mit Prüffrequenzen im für die Werkstoffprüfung und den Medizinbereich interessanten MHz-Bereich.

Fig. 2 bis 9 geben Gestaltungsbeispiele der Erfindung in verschiedenen Ansichten wieder.

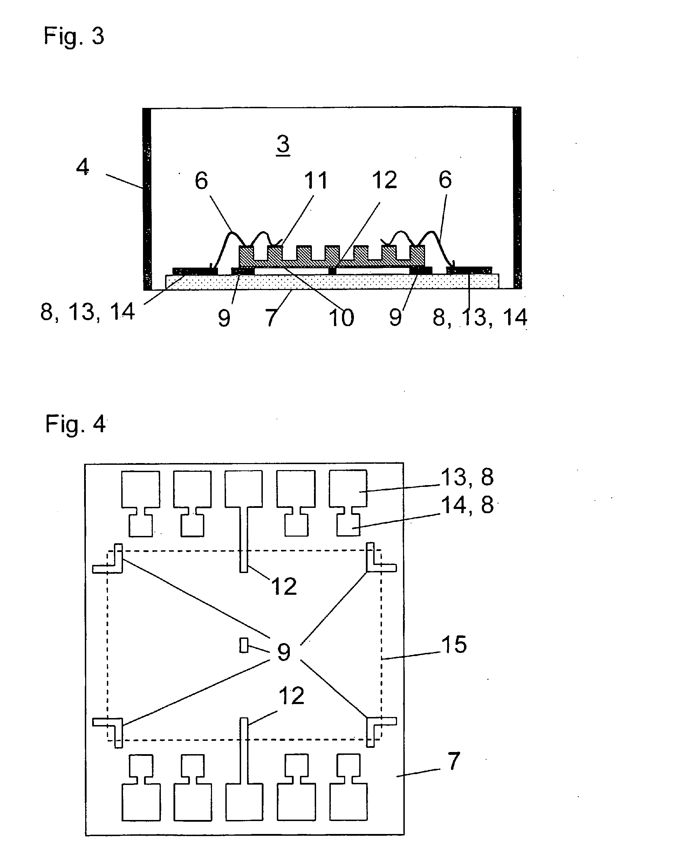

Fig. 2 und 3 zeigen dabei je eine seitliche Schnittansicht durch einen Ultraschallwandler, umfassend eine Platine 7, mit aufgebrachter Leiterbahnstruktur und einem auf dieser aufgesetzten piezoelektrischen Körper 1 (Fig. 2) oder Wandlerarray 11 (Fig. 3). Fig. 4 gibt in einer Aufsicht das Design dieser Leiterbahnstruktur auf der Platine 7 wieder. Die Leiterbahnstruktur auf der Platine umfasst außerhalb des Überdeckungsbereichs 15 des Körpers 1 oder Wandlerarrays 11 mehrere Kontaktpads 8 sowie innerhalb des Überdeckungsbereichs 15 mehrere Abstandshalter 9 ohne elektrische Funktion und zwei Massekontakte 12 als elektrische Kontaktierung für die untere Elektrode des Wandlers oder Wandlerarrays. Der Ultraschallwandler ist in ein Gehäuse 4 eingesetzt und ist dort im Dämpfungskörper 3 eingegossen.

Der piezoelektrischen Körper oder Wandler weist zwei planparallel zueinander angeordnete, in vorgenannte Weise metallisierte Elektrodenflächen (obere und untere Elektrode) auf und ist in den in Fig. 2 dargestellten Ausführungsbeispiel quaderförmig. Ein solcher piezoelektrischer Körper schwingt bei einer elektrischen Anregung in drei Grundfrequenzen, welche durch die Abmessungen des Quaders vorgegeben sind. Zur Vermeidung von überlagerten Schwingungen ist dieser Effekt bei der Dimensionierung des piezoelektrischen Körpers zu berücksichtigen.

Ein in Fig. 3 dargestellter Wandler besteht dagegen aus einer größeren Anzahl von kleinen Säulen oder Streifen, welche aus einem piezoelektrischen Körper durch Drahtsägen herausgearbeitet sind und mit einer Vergussmasse zu einer Platte oder Scheibe vergossen sind. Im Ausführungsbeispiel wurde eine maximale Dicke der Säulen deutlich keiner als die halbe Wellenlänge λ/2 im piezoelektrischen Material des Wandlers gewählt. Ein solcher Wandler besitzt im Wesentlichen nur ein Schwingungsmode, nämlich die Dickenschwingung, während sich die vergleichsweise hohen radialen Resonanzfrequenzen durch die Vergussmasse wirksam durch Dämpfung unterdrückbar sind.

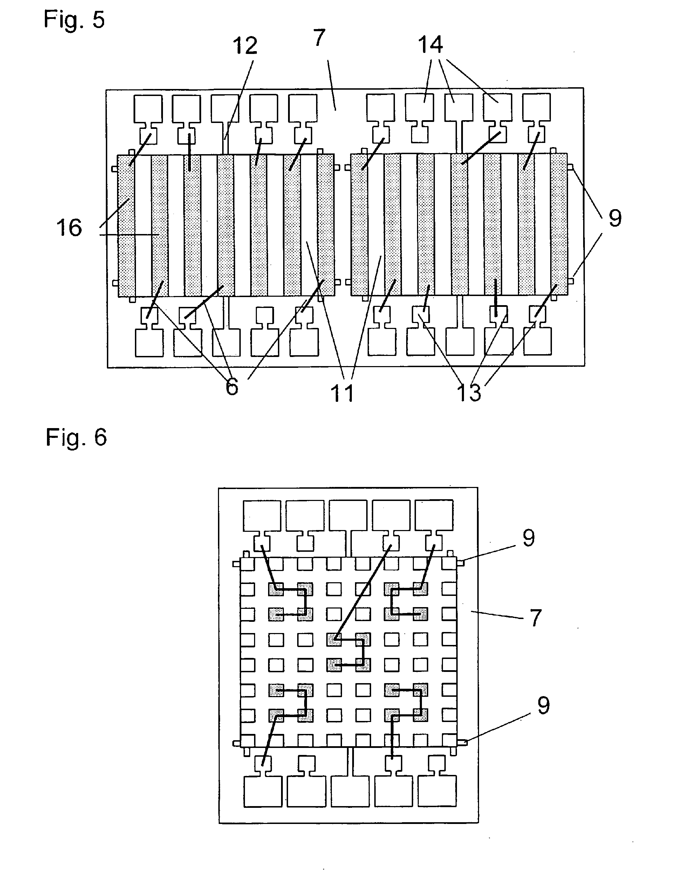

Die Leiterbahnen sind als Leiterbahnstruktur aus einer auf einem Platinenrohling aufgebrachte Kupferschicht herausgeätzt (übliche Ätztechnik mit Photolack und Maske). Die Leiterbahnhöhe entspricht somit der Dicke der ursprünglichen Kupferschicht, wodurch eine einheitliche Höhe der gesamten Leiterbahnstruktur sichergestellt ist, und zwar mit der hohen Fertigungsgenauigkeit der geringen Toleranzbreiten von 10 % der aufgebrachten Kupferschicht. Zwischen piezoelektrischen Körper 1 bzw. dem Wandlerarray 11 und der Platine 7 erstreckt sich, wie in Fig. 4 dargestellt, im Überdeckungsbereich 15 ein Klebespalt 10 mit eben der exakt einhaltbaren Dicke, die der Leiterbahnstrukturhöhe entspricht. Kontaktpads 8 die eine elektrische Verbindung (Leiterbahn) zu den Massekontakten 12 aufweisen, dienen der Kontaktierung der unteren Elektrode, während andere Kontaktpads, die die genannten elektrischen Verbindungen nicht aufweisen, für gebondete (z.B. über Ültraschallbonden oder Punktverschweißen) Drahtverbindungen zu den oberen Elektroden zur Verfügung stehen (vgl. Fig. 2 und 3). Dabei weisen die Kontaktpads 8 eine kleinere Bondingfläche 13 und eine mit dieser elektrisch verbundene größere Lötfläche 14 auf (vgl. auch Fig. 5).

Fig. 4 zeigt die Position der Abstandshalter 9 unter dem Überdeckungsbereich 15. Während sechs dieser Abstandshalter im Randbereich positioniert sind, befindet sich ein weiterer zentral in der Mitte. Ein zentraler Abstandshalter 9 dient der zusätzlichen Stütze und ist vor allem dann erforderlich, wenn der aufgesetzte piezoelektrische Körper nach dem Aufkleben mechanisch, beispielsweise mit einer Drahtsäge zu einem Arraywandler gemäß Fig. 3, bearbeitet wird oder das Verhältnis aus laterale Erstreckung zu Dicke des piezoelektrischen Körpers eine gewisse Größe überschreitet. Er ist aber so anzuordnen, dass das durch die Ankopplungsschicht hindurch geführte Ultraschallsignal nicht oder nur unwesentlich beeinflusst wird. Im vorliegenden Fall ist der zentrale Abstandshalter mittig im Schallfeld angeordnet, d. h. eine Beeinflussung eines Ultraschallsignals erfolgt ohne eine bestimmte Vorzugsrichtung.

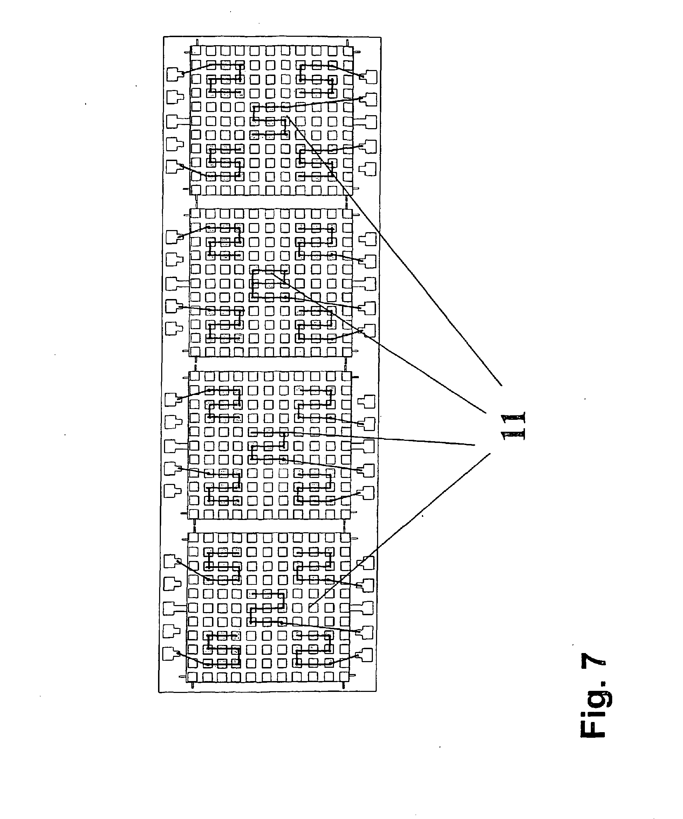

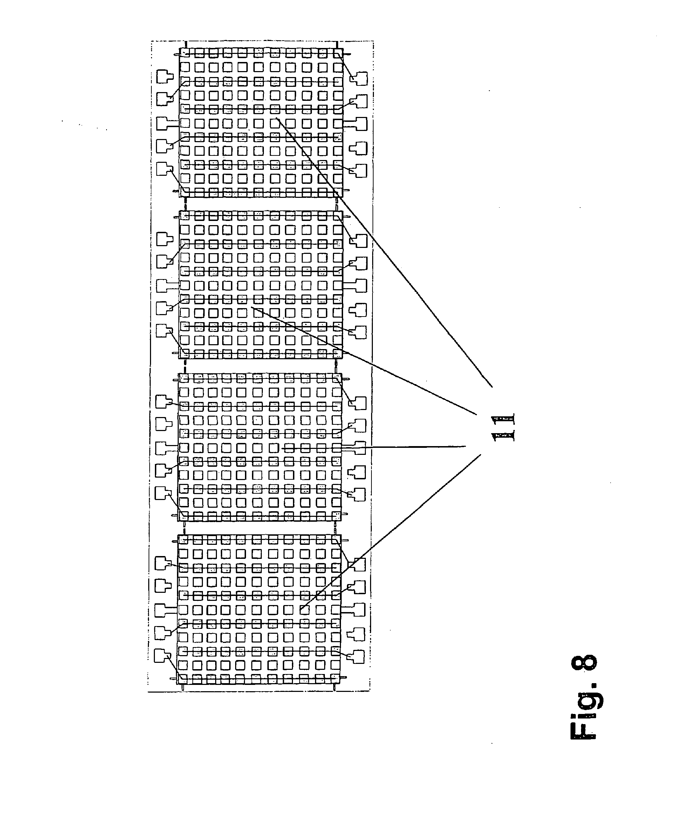

Eine Aufsicht von zwei streifenförmig strukturierten Wandlerarrays 11 auf einer gemeinsamen Platine 7 ist in Fig. 5 dargestellt. Fig. 6 bis 8 zeigen dagegen säulenförmig strukturierte Wandlerarrays auf einer Platine 7. Hierbei sind jeweils eine gewisse Anzahl von Einzelelementen zu Einzelelementgruppen zusammengeschaltet ist. Durch Aneinanderreihen von mehreren Arraywandler auf einer gemeinsamen Leiterplatte lassen sich zudem Ultraschallwandler auch größerer lateralen Erstreckungen oder auch mit Krümmungen realisieren, die mit einer geeigneten Elektronik versehen ein Ultraschall-Phasen-Array bilden (Fig. 7 und 8). Da die Bruchgefahr des piezoelektrischen Körpers bei vorgegebener Dicke mit der lateralen Ausdehnung steigt, bietet sich im Sinne einer Reduzierung der Bruchgefahr bei Applizierung der Körper oder auch im Sinne einer Vermeidung von zentral im Überdeckungsbereich angeordneter Abstandshalter eine Anordnung mehrerer piezoelektrischer Körper (oder Wandlerarrays) nebeneinander auf einer gemeinsamen Platine an, wobei die Körper elektronisch gemeinsam zu einem Ultraschallwandler verschaltet werden.

Durch elektrisches Zusammenschalten von mehreren Säulen kann prinzipiell im Rahmen eines durch die Säulen vorgegebenen Rasters praktisch jede beliebige aktive Wandlergruppenerstreckung (Sensorform) erzeugt werden. Fig. 6 und 7 zeigen je eine schachbrettförmige Erstreckung von Säulengruppen mit aus einer Erstreckung jedes Körpers (Wandlerarray) von ca. 5 * 5 mm, wobei jeweils 9 Säulen zu einer Säulengruppe verschaltet sind. Durch Aneinanderreihen von mehreren Wandlerarrays lassen sich beliebig große Sensorfelder aufbauen (Fig. 7). Bei einer phasenversetzten Ansteuerung einzelner Säulengruppen ist es möglich die Schallausbreitung eines Ultraschallimpulses (Schallkeule des Ultraschallsensors) zu beeinflussen.

Fig. 8 zeigt einen Ultraschalwandler, welcher bis auf eine streifenförmige Zusammenschaltung von Säulengruppen in Streifenform der Ausführungsform gemäß Fig. 7 entspricht. Durch die Strukturierung und Zusammenschalten der einzelnen Säulen wird eine deutlich Verbesserung der Sensoreigenschaften im Gegensatz zu der Streifengeometrie erreicht.

Die Säulengruppen sind auf der oberen Elektrode mit Drahtverbindungen (elektrische Anschlüsse 6) kontaktiert. Die Drahtverbindung darf die akustischen Eigenschaften nicht oder nur unwesentlich beeinflussen. Kontaktierungen durch Löten oder Leitkleben scheiden aufgrund der geringen lateralen Ausdehnung der Säulen oder der thermischen Belastung des piezoelektrischen Materials aus. Neuere Techniken bevorzugen leitfähige Folien die unter Druck und durch Verkleben einen elektrischen Kontakt herstellen.

Im Rahmen des Ausführungsbeispiels erfolgt die Kontaktierung der oberen Elektroden auf den Säulen durch ein Ultraschall-Drahtbondverfahren. Die thermischen Einflüsse sind lokal begrenzt und sehr gering da die Drahtdicken mit 30 µm - 70 µm noch klein im Verhältnis zu den Elektrodenflächen z.B. ca. 300 µm * 300 µm der Säulen sind. Der Bondprozess kann kalt durchgeführt werden kann und die Piezokeramik wird thermisch praktisch nicht beansprucht. Kontaktiert wird von der Piezosäulen direkt zu den Leiterbahnen (Bondingflächen) oder auch wie in Fig. 6 bis 8 dargestellt von Säule zu Säule mit nur einer Verbindung zur Leiterbahn. Der Anschluss an die nachfolgende Elektronik erfolgt über geeignete wesentlich dickere elektrische Leitungen von der Leiterbahn (Lötflächen) aus.

Die Herstellung eines Ultraschallwandlers umfasst die folgenden einzelnen Verfahrensschritte:

Im Rahmen der Herstellung dieser Ausführungsformen wird in einem Arbeitsschritt ein piezoelektrischer Körper 1 mit einem schnell aushärtenden niederviskosen Klebstoff (Sekundenkleber auf Cyancrylat-Basis) auf eine Platine 7 aufgeklebt, wobei die Abstandshalter 9 und die Massekontakte 12 eine Dicke des Klebespalts 10 vorgeben (vgl. Fig. 2 bis 4). Wichtig ist, dass der Klebstoff blasenfrei aushärtet, um eine akustische Kopplung zwischen Platine 7 und piezoelektrischem Körper 1 sicherzustellen. Dazu wird in der Mitte des Überdeckungsbereichs 15 (Auflagefläche) ein Tropfen des vorgenannten Klebstoffs aufgebracht, der bei dem Aufsetzen des piezoelektrischen Körpers sich zur Seite hin gleichmäßig und blasenfrei verteilt. Die Abstandshalter 9 dürfen diesen Verdrängungsvorgang nicht behindern, d.h. sie sind so zu gestalten, dass dieser Fluss nicht unterbrochen wird und sich Luftkammern bilden können. Die Abstandshalter gemäß Fig. 3 und 4 befinden sich am äußeren Rand oder an solchen Stellen, die akustisch nicht oder im eingangs genannten Sinne nur wenig aktiv sind oder durchschallt werden. Da die Dicke des Klebespalts 10 im vorliegenden Beispiel mit 30 µm erheblich kleiner als die Wellenlänge des akustischen Ultraschallsignals bei 3 MHz ist, ist der akustische Einfluss Klebschicht auf die Wandlereigenschaften vernachlässigbar.

Beim Anpressen des piezoelektrischen Körpers oder Wandlers auf die Abstandshalter 9 und die Massekontakte 12 entsteht eine elektrisch leitende Verbindung der unteren Elektrode des piezoelektrischen Körpers oder Wandlers und den Massekontakten 12. Die elektrisch leitende Verbindung lässt sich in besonders vorteilhafter Weise über den gesamten Herstellungsprozess über eine Kontaktwiderstandsmessung zwischen zwei Massekontakten, welche elektrisch über die untere Elektrode kurzgeschlossen ist, überprüfen. Ein kleiner Widerstandswert entspricht einer guten Kontaktierung und ist während des Fertigungsprozesses leicht kontrollierbar. Der Vorteil dieser Methode besteht darin, dass die benötigte Kraft relativ gering gehalten werden kann, was die Gefahr eines Zerbrechens des piezoelektrischen Körpers reduziert. Eine geringere Kraft bewirkt auch, dass bei einer Entlastung des Verbunds nach einem Aushärten des Klebstoffs ein geringeres inneres Spannungsniveau zurückbleibt. Die reduziert auch ein Aufreißen der Klebverbindung beispielsweise bei Temperaturwechselbelastungen oder im Rahmen von anschließenden mechanischen Bearbeitungsschritten, welche wiederum einen Kontaktverlust und damit einen Ausfall des Ultraschallwandlers bewirken können. Diese Nachteile und Einschränkungen werden durch die beschriebenen Verfahrensschritte unter Verwendung der Abstandshalter und Massekontakte vermieden. Zudem wird eine hohe Reproduzierbarkeit der Ultraschallwandler in Bezug auf ihre akustischen und elektrischen Eigenschaften erzielbar.

Außerdem würden Drahtverbindungen mit den vorgenannten Abmessungen zu der unteren Elektrode anstelle der vorgenannten Massekontakte bei Ultraschallwandlern insbesondere mit lateralen Abmessungen unter ca. 2 mm und Dicken unter ca. 0,4 mm zusätzliche Schwingungsmoden, die einen Einsatz als Ultraschallwandler zusätzlich beeinflussen.

Der Anpressvorgang wird mit dem Aushärten des Klebers abgeschlossen. Eine Strukturierung der vorgenannten Art des piezoelektrischen Körpers erfolgt nach dem Aushärten, wobei eine qualitativ hochwertige und zuverlässige, aber kostengünstige Strukturierung des piezoelektrischen Körper in vorteilhafter Weise mit der Platine als stabilen, in der Strukturierungsvorrichtung fixierten Träger erfolgt.

Die Piezoelemente werden bevorzugt mit einer Drahtsäge (Wafersäge) strukturiert, wobei die Schnitttiefe sich nicht über die gesamte Dicke des piezoelektrischen Körpers erstreckt, sondern bevorzugt eine Restdicke von kleiner λ/8 sowie die untere Elektrodenfläche unstrukturiert bestehen bleibt. Der Einfluss dieser unstrukturierten Restdickenbereiche ist für das Impuls-Echo-Verfahren nahezu vernachlässigbar.

Es folgt ein Kontaktieren oder obere Elektrode auf vorgenannter Weise. Im Anschluss daran wird der vollständige Ultraschallwandler mit Elektronik in einem Gehäuse vergossen. Damit wird die akustische Dämpfung 3 des Ultraschallsensors eingestellt, die Bonddrähte 13 vor mechanischen Belastungen geschützt und das ganze System vor äußeren Einflüssen unempfindlich gemacht.

Alle beschriebenen Verfahrensschritte lassen sich mit Geräten der modernen SMT und Dickfilmtechnik zur zuverlässigen und kostengünstigen Herstellung der genannten Ultraschallwandler automatisch durchführen. Die so gefertigten Ultraschallwandler weisen eine besonders gute Reproduzierbarkeit ihrer elektrischen und akustischen Eigenschaften auf, was gerade für Systeme, die mit einer großen Anzahl von Sensoren ausgestattet sind, von hoher Bedeutung ist.

Der Prozess lässt sich vollständig automatisieren. Des Weiteren können dann auf dieser Platine gleichzeitig die benötigten elektronischen Baugruppen wie Vorverstärker und Sendestufen mit integriert und bestückt werden. Durch die kurze Entfernung von Ultraschallwandler zu Elektronik sind elektrische Störeinflüsse sehr gering. Die Qualität der Ultraschallsignale lassen sich damit erheblich verbessern. Durch diese einfache Integration von Ultraschallwandler und Elektronik können die Systeme sehr viel kleiner gebaut und Kosten reduziert werden.

### Literatur:

[1] EP 1145772 A2
[2] DE 100 50 232 A1
[3] R. Stotzka, H. Widmann, T. Müller, and K. Schlote-Holubek: Prototype of a new 3D ultrasound computer tomography system: transducer design and data recording; Vortrag am 18.Februar 2004 im SPIE's Internl. Symposium Medical Imaging 2004 (14.-19.Februar 2004 in San Diego, USA)

### Bezugszeichenliste

- 1: Wandler, piezoelektrischer Körper
- 2: Anpassungsschicht
- 3: Dämpfungskörper
- 4: Gehäuse
- 5: Elektroden
- 6: Elektrische Anschlüsse
- 7: Platine
- 8: Kontaktpad
- 9: Abstandshalter
- 10: Klebspalt
- 11: Wandlerarray
- 12: Massekontakt
- 13: Bondingfläche
- 14: Lötfläche
- 15: Überdeckungsbereich
- 16: Bondingdrähte

## Patentansprüche

1. Ultraschallwandler, umfassend
a) mindestens einen piezoelektrischen Körper (1, 11) mit jeweils mindestens einer unteren und einer oberen Elektrode (5) sowie
b) eine Ankopplungsschicht, umfassend mindestens eine Platine (7), auf die der Ultraschallwandler mit der unteren Elektrode verbunden ist,
**dadurch gekennzeichnet, dass**
c) auf den Platinen eine Leiterbahnstruktur (9, 12) mit konstanter Leiterbahnhöhe unter den piezoelektrischen Körpern vorgesehen ist, welche in einem elektrischen Kontakt mit der unteren Elektrode steht sowie
d) der verbleibende Hohlraum (10) zwischen Platine und piezoelektrischem Körper neben der Leiterbahnstruktur vollständig durch ein Bindemittel ausgefüllt ist.

2. Ultraschallwandler nach Anspruch 1, **dadurch gekennzeichnet, dass** die Leiterbahnstruktur sich nur unter Bereiche der piezoelektrischen Körper erstreckt, welche akustisch wenig oder nicht aktiv sind.

3. Ultraschallwandler nach Anspruch 2, **dadurch gekennzeichnet, dass** die Bereiche durch die Randbereiche der piezoelektrischen Körper gebildet werden.

4. Ultraschallwandler nach einem der Ansprüche 1 bis 3, **dadurch gekennzeichnet, dass** die Platinen eine akustische Impedanz aufweist, welche zwischen der der piezoelektrischen Körper und der des Wassers liegt.

5. Ultraschallwandler nach einem der Ansprüche 1 bis 4, **dadurch gekennzeichnet, dass** das Bindemittel ein Klebstoff ist.

6. Ultraschallwandler nach Anspruch 5, **dadurch gekennzeichnet, dass** in dem Klebstoff Partikel eines Füllkörpermaterials eingemischt sind, welche die akustische Impedanz und das Dämpfungsverhalten des Klebstoffs beeinflusst.

7. Ultraschallwandler nach einem der Ansprüche 1 bis 6, **dadurch gekennzeichnet, dass** der piezoelektrische Körper eine Strukturierung in der Form von Vertiefungen ausgehend von der oberen Elektrode aufweist, welche sich nicht auf die untere Elektrode erstrecken.

8. Ultraschallwandler nach Anspruch 7, **dadurch gekennzeichnet, dass** sich die Vertiefungen ausschließlich zu mindestens 80% des Abstandes von oberer zu unterer Elektrode erstrecken.

9. Ultraschallwandler nach Anspruch 7 oder 8, **dadurch gekennzeichnet, dass** die Vertiefungen Sägeschnitte sind, welche Säulen- oder Streifenstrukturen umgrenzen.

10. Verfahren zur Herstellung von Ultraschallwandlern, umfassend die folgenden Verfahrensschritte
a) Bereitstellung mindestens eines piezoelektrischen Körpers mit jeweils mindestens einer unteren und einer oberen Elektrode sowie mindestens einer Platine als Ankopplungsschicht,
b) Auftragen eines Bindemittels auf die Platinen sowie
c) Aufsetzen und Anpressen der piezoelektrischen Körper jeweils mit der unteren Elektrode auf das Bindemittel, welches durch das Anpressen sich seitlich über die gesamte untere Elektrode ausbreitet,
**dadurch gekennzeichnet, dass**
d) auf den Platinen eine Leiterbahnstruktur mit konstanter Leiterbahnhöhe im Bereich unter dem piezoelektrischen Körper aufgebracht ist sowie
e) durch das Anpressen ein elektrischer Kontakt zwischen Leiterbahnstruktur und unterer Elektrode entsteht, wobei das Bindemittel den verbleibenden Hohlraum zwischen Platine und den piezoelektrischen Körpern neben der Leiterbahnstruktur vollständig ausfüllt.

11. Verfahren nach Anspruch 10, **dadurch gekennzeichnet, dass** das Bindemittel einen Klebstoff umfasst, welcher als Tropfen zwischen die Leiterbahnstruktur in der Mitte des Bereichs aufgetragen wird.

12. Verfahren nach Anspruch 11, **dadurch gekennzeichnet, dass** das Anpressen des piezoelektrischen Körpers mit dem Aushärten des Klebstoffs beendet ist.

13. Verfahren nach einem der Ansprüche 10 bis 12, umfassend einen weiteren Verfahrensschritt:
Überprüfung des elektrischen Kontakts mit einer Widerstandsmessung zwischen zwei von einander getrennten Leiterbahnstrukturen, welche von einer unteren Elektrode überbrückt werden.

14. Verfahren nach einem der Ansprüche 10 bis 13, umfassend einen weiteren Verfahrensschritt:
Mechanische Strukturierung der auf den Platinen fixierten piezoelektrischen Körper von der zweiten Elektrode aus, wobei sich die Strukturierung nicht auf die untere Elektrode erstreckt.

15. Verfahren nach einem der Ansprüche 10 bis 14, umfassend einen weiteren Verfahrensschritt:
Schaffung von mindestens einem elektrischen Anschluss auf der oberen Elektrode mit einem Ultraschalldrahtbondverfahren.

## Claims

1. Ultrasonic transducer, including
a) at least one piezoelectric body (1, 11) having respectively at least one lower and one upper electrode (5), and
b) a coupling layer, including at least one plate (7), on which the ultrasonic transducer is connected to the lower electrode,
**characterised in that**
c) a conductor path structure (9, 12), having a constant conductor path height beneath the piezoelectric bodies, is provided on the plates and is in electrical contact with the lower electrode, and
d) the remaining cavity (10) between plate and piezoelectric body adjacent the conductor path structure is completely filled by a bonding agent.

2. Ultrasonic transducer according to claim 1, **characterised in that** the conductor path structure only extends beneath regions of the piezoelectric bodies which are little or not at all active acoustically.

3. Ultrasonic transducer according to claim 2, **characterised in that** the regions are formed by the edge regions of the piezoelectric bodies.

4. Ultrasonic transducer according to one of claims 1 to 3, **characterised in that** the plates have an acoustic impedance which lies between that of the piezoelectric bodies and that of water.

5. Ultrasonic transducer according to one of claims 1 to 4, **characterised in that** the bonding agent is an adhesive.

6. Ultrasonic transducer according to claim 5, **characterised in that** particles of a filling body material are mixed into the adhesive and influence the acoustic impedance and the damping behaviour of the adhesive.

7. Ultrasonic transducer according to one of claims 1 to 6, **characterised in that** the piezoelectric body has a structuring in the form of indentations which emanate from the upper electrode but do not extend to the lower electrode.

8. Ultrasonic transducer according to claim 7, **characterised in that** the indentations extend exclusively as far as at least 80 % of the spacing from the upper to the lower electrode.

9. Ultrasonic transducer according to claim 7 or 8, **characterised in that** the indentations are saw cuts, which define column or strip structures.

10. Method of producing ultrasonic transducers, said method including the following methods steps:
a) preparing at least one piezoelectric body with respectively at least one lower and one upper electrode, and at least one plate as the coupling layer,
b) applying a bonding agent to the plates, and
c) placing the piezoelectric bodies respectively with the lower electrode onto the bonding agent and pressing them thereon, the bonding agent spreading laterally over the entire lower electrode through the pressing-on action,
**characterised in that**
d) a conductor path structure is applied on the plates with a constant conductor path height in the region beneath the piezoelectric body, and
e) an electrical contact between the conductor path structure and lower electrode is produced through the pressing-on action, the bonding agent completely filling the remaining cavity between the plate and the piezoelectric bodies adjacent the conductor path structure.

11. Method according to claim 10, **characterised in that** the bonding agent includes an adhesive which is applied as drops between the conductor path structure in the centre of the region.

12. Method according to claim 11, **characterised in that** the pressing-on of the piezoelectric body is terminated with the hardening of the adhesive.

13. Method according to one of claims 10 to 12, including an additional method step:
checking the electrical contact with a resistance measurement between two conductor path structures which are separated from each other and are spanned by a lower electrode.

14. Method according to one of claims 10 to 13, including an additional method step:
mechanical structuring of the piezoelectric bodies, secured on the plates, from the second electrode, the structuring not extending to the lower electrode.

15. Method according to one of claims 10 to 14, including an additional method step:
creating at least one electrical connection on the upper electrode with an ultrasonic wire bonding method.

## Revendications

1. Convertisseur d'ultrasons comprenant :
a) au moins un corps piézo-électrique (1, 11) ayant au moins une électrode inférieure et une électrode supérieure (5) ainsi que
b) une couche de couplage comprenant au moins une platine (7) à laquelle est relié le convertisseur d'ultrasons par son électrode inférieure,
**caractérisé en ce que**
c) une structure de chemins conducteurs (9, 12) de hauteur de chemins conducteurs constante est prévue sur les platines sous les corps piézo-électriques en étant en contact électrique avec l'électrode inférieure, et
d) la cavité (10) qui reste entre la platine et le corps piézo-électrique à côté de la structure des chemins conducteurs, est complètement remplie d'un liant.

2. Convertisseur d'ultrasons selon la revendication 1,
**caractérisé en ce que**
la structure de chemins conducteurs s'étend seulement sous les zones du corps piézo-électrique qui ne sont que peu ou pas actives du point de vue acoustique.

3. Convertisseur d'ultrasons selon la revendication 2,
**caractérisé en ce que**
les zones sont formées par les zones marginales du corps piézo-électrique.

4. Convertisseur d'ultrasons selon l'une des revendications 1 à 3,
**caractérisé en ce que**
les platines ont une impédance acoustique comprise entre celle des corps piézo-électriques et l'eau.

5. Convertisseur d'ultrasons selon l'une des revendications 1 à 4,
**caractérisé en ce que**
le liant est un adhésif.

6. Convertisseur d'ultrasons selon la revendication 5,
**caractérisé en ce que**
l'adhésif contient des particules en une matière de charge, mélangées, influençant l'impédance acoustique et le comportement d'amortissement de l'adhésif.

7. Convertisseur d'ultrasons selon l'une des revendications 1 à 6,
**caractérisé en ce que**
le corps piézo-électrique a une structure sous la forme de cavités partant de l'électrode supérieure et n'atteignant pas l'électrode inférieure.

8. Convertisseur d'ultrasons selon la revendication 7,
**caractérisé en ce que**
les cavités s'étendent exclusivement jusqu'à environ 90 % de la distance séparant l'électrode supérieure et l'électrode inférieure.

9. Convertisseur d'ultrasons selon la revendication 7 ou 8,
**caractérisé en ce que**
les cavités sont des traits de scie délimitant des structures en colonnes ou en rangées.

10. Procédé de fabrication de convertisseur d'ultrasons comprenant les étapes suivantes :
a) fourniture d'au moins un corps piézo-électrique ayant chaque fois au moins une électrode inférieure et une électrode supérieure et au moins une platine comme couche de couplage ;
b) application d'un liant sur la platine ; et
c) mise en place et compression des corps piézo-électriques chaque fois avec l'électrode inférieure contre le liant qui, par compression, s'étale latéralement sur toute l'électrode inférieure,
**caractérisé en ce que**
d) on applique une structure de chemins conducteurs sur les platines, structure de hauteur de chemins conducteurs constante, dans la zone située sous le corps piézo-électrique ; et
e) par compression on forme un contact électrique entre la structure de chemin conducteur et l'électrode inférieure, le liant remplissant complètement l'espace subsistant entre la platine et les corps piézo-électriques à côté de la structure de chemins conducteurs.

11. Procédé selon la revendication 10,
**caractérisé en ce que**
le liant comporte un adhésif que l'on applique sous la forme de gouttes entre la structure de chemins conducteurs au milieu de la zone.

12. Procédé selon la revendication 11,
**caractérisé en ce qu'**
on termine la compression du corps piézo-électrique par la prise de l'adhésif.

13. Procédé selon l'une des revendications 10 à 12 comprenant une autre étape :
contrôle du contact électrique par une mesure de résistance électrique entre deux structures de chemins conducteurs séparées l'une de l'autre et qui sont chevauchées par une électrode inférieure.

14. Procédé selon l'une des revendications 10 à 13 comprenant une autre étape de procédé :
mise en structure mécanique des corps piézo-électriques fixés sur les platines pour la seconde électrode, la mise en structure n'atteignant pas l'électrode inférieure.

15. Procédé selon l'une des revendications 10 à 14 comprenant une autre étape de procédé :
création d'au moins un branchement électrique sur l'électrode supérieure par un procédé de liaison de fils par ultrasons.
